# EUROPEAN PATENT SPECIFICATION

(11) **EP 4 504 136 B1**
(45) Date of publication and mention of the grant of the patent: **15.10.2025**
(21) Application number: 23717078.2
(22) Date of filing: 30.03.2023
(51) Int. Cl.: A61K 8/34, A61K 8/36, A61K 8/44, A61Q 15/00, A45D 40/00, A61K 8/02

(54) **DEODORANT STICK PRODUCTS**
DEODORANTSTIFTPRODUKTE
PRODUITS EN STICK DE DÉODORANT

(30) Priority: 04.04.2022 EP 22166527
(43) Date of publication of application: 12.02.2025
(73) Proprietor: Unilever IP Holdings B.V., 6708 WH Wageningen (NL); Unilever Global IP Limited, Wirral, Merseyside CH62 4ZD (GB)
(72) Inventor: ALVAREZ, Sebastian, 6708 WH Wageningen (NL); BASKERVILLE, Tammy, 6708 WH Wageningen (NL); MONPOU TRUCHET, Caroline, 6708 WH Wageningen (NL)
(74) Representative: Turner, Felicity Margaret Mary
(86) International application number: PCT/EP2023/058411
(87) International publication number: WO 2023/194216

(56) References cited:
- EP-A2- 0 284 765
- WO-A1-2020/152223
- US-A1- 2010 254 926
- US-A1- 2010 272 665
- US-A1- 2020 323 754

## Description

### Field of the Invention

The present invention is in the field of deodorant stick products and their method of manufacture. It is particularly concerned with refillable deodorant stick products that are suitable for use in the underarm regions of the human body.

### Background of the Invention

Numerous refillable deodorant stick products have been disclosed in the prior art and are available on the market.

WO 2020/152,224 A1 (Unilever, 2020) discloses a refillable stick product whereby the refill unit is reversibly connected to a holder via a tongue and groove mechanism. This publication does not give details of stick compositions suitable for use with the hardware claimed.

WO 2021/078,715 A1 (Unilever, 2021) discloses a refillable stick product whereby the refill unit is reversibly connected to a holder via a bayonet and socket mechanism. This publication is again silent concerning details of suitable stick compositions.

WO 2020/152,223 A1 (Unilever, 2020) discloses a refillable stick product wherein a deodorant stick composition is held on a retaining member by arcuate bridge structures that arc into the composition and become embedded therein. Again, this publication does not give details of suitable stick compositions.

Numerous problems are associated with refillable deodorant stick products. First, during the refilling process the stick composition, and often an associated retaining member, has to be manually engaged with the holder. This process inevitably places stresses upon the stick composition and any associated retaining member. There can be a tendency for the stick composition to shear off from its retaining member unless it is firmly affixed.

A second problem can arise during application of refillable deodorant stick products. Because such products are typically drawn across the surface of the skin, lateral forces are exerted on the stick composition which can again cause it to shear off from its retaining member. This problem, and the one referred to in the paragraph immediately above, are exacerbated when the refillable deodorant stick product does not have a spindle running through its centre. Such spindles are present in more conventional deodorant stick products and serve to reinforce the composition on its retaining member or platform.

The problems referred to in the two paragraphs immediately above have been addressed to an extent by hardware features as described in some of the publications referred to above. The present invention addresses these problems via suitable selection of the deodorant stick composition.

Many prior art deodorant stick compositions are glycol-based and use a sodium salt of a fatty acid as a structurant. The following publications describe such products.

EP 3,445,324 B1 (Unilever, 2019) discloses deodorant stick compositions comprising dipropylene glycol (DPG), propylene glycol (PG), glycerol, water and a structurant. The use of such compositions in refillable deodorant stick products is not disclosed.

WO 2021/239,360 A1 (Beiersdorf, 2021) discloses PEG-free soap gel compositions comprising linear fatty acids in the form of soaps, 1,3-propanediol, glycerol and butylene glycol. Paragraph [0021] of the description states that "The liner fatty acids are present predominately as alkali salts (alkali soaps), preferably as sodium soap... However, a small proportion of free, unsaponified fatty acids, about in an amount of 0.01 to 0.5 wt. percent of the preparation, is not disadvantageous". Again, there is no disclosure of such compositions being used in refillable deodorant stick products.

### Summary of the Invention

It is an object of the present invention to provide a refillable deodorant stick product that has enhanced retention of the deodorant stick composition on an associated retaining member. As mentioned above, refillable deodorant stick products, particularly those without a central spindle, are prone to lateral forces being exerted on the stick composition which can cause it to shear off from its retaining member.

The problem of breakage or shearing of the deodorant composition from its retaining member is also exacerbated when the stick has an oval cross-section. Deodorant stick products having an oval cross-section are desirable because they are aesthetically pleasing; however, consumers typically tend to apply such compositions by drawing the deodorant composition across the skin in a direction orthogonal to the longer axis of the oval. This increases the likelihood that the lateral stress developed during topical application will cause breakage.

The stress problem associated with topical application of refillable deodorant stick products is also exacerbated by the fact that such products are applied multiple times. Whilst the product may survive the first few applications, repeated application increases the likelihood of failure, i.e. stick breakage.

It is a further object of the present invention to provide a refillable deodorant stick product that has uncompromised pay-out, i.e. uncompromised delivery of the deodorant stick composition onto the surface to which it is applied.

It is a further object of the present invention to provide a refillable deodorant stick product that comprises a deodorant stick composition having uncompromised hardness. It is preferred that the deodorant stick composition has a hardness of at least 600 gram force. In terms of the standard cone penetration test described further herein, it is preferred that the deodorant stick composition has a hardness of less than 10 mm, more preferably less than 9 mm and most preferably less than 8 mm.

In a first aspect of the invention, there is provided a refillable deodorant stick product comprising:
- a deodorant stick composition mounted on an axially immobile retaining member,
- the retaining member being reversibly connectable to a holder enabling the deodorant stick product to be held in the human hand,
- wherein elements of the retaining member are embedded in the deodorant stick composition,
- wherein the deodorant stick composition comprises:
   i. at least 50 wt.% of one or more glycols selected from the group consisting of propylene glycol, propanediol, dipropylene glycol, butylene glycol, glycerol;
   ii. 5 to 40 wt.% water;
   iii. 1 to 20 wt.% of sodium salt of one or more C14-C22 fatty acids,
characterised in that the molar ratio of free fatty acid to sodium salt of fatty acid is less than 1: 99.

In a second aspect of the invention, there is provided a process for the manufacture of a refillable deodorant stick product, the product comprising:
- a deodorant stick composition mounted on an axially immobile retaining member,
- the retaining member being reversibly connectable to a holder enabling the deodorant stick product to be held in the human hand,
- wherein elements of the retaining member become embedded in the deodorant stick composition;
wherein the process comprises the steps of:
a. combining as a flowable liquid:
   (i) at least 50 wt.% of one or more glycols selected from the group consisting of propylene glycol, propanediol, dipropylene glycol, butylene glycol, glycerol;
   (ii) 5 to 40 wt.% water;
   (iii) 1 to 20 wt.% of sodium salt of one or more C14-C22 fatty acids,
b. adding a base in an amount such that the molar ratio of the base to the sodium salt of a fatty acid is from 2: 98 to 25: 75,
c. pouring the flowable liquid produced in the above steps into a mould such that elements of the retaining member are covered by the flowable liquid, and
d. cooling the flowable liquid such that it forms a solid deodorant stick composition attached to the retention member and with elements of the retention member embedded in the solid deodorant stick composition.

In a third aspect of the invention, there is provided a process according to second aspect of the invention for the manufacture of a refillable deodorant stick product according to the first aspect of the invention.

In a fourth aspect of the invention, there is provided a cosmetic method of reducing malodour comprising the use of a refillable deodorant stick product according to the first aspect of the invention to topically apply deodorant stick composition to the surface of the human body.

### Detailed Description

Herein, cosmetic methods and uses should be understood to be non-therapeutic methods.

Herein, all percentages, parts, and ratios are by weight, unless otherwise indicated.

Herein, references to an amount of a material or materials refer to the total amount of material(s) of the type indicated.

Herein, references to an amount of a material are with reference to the total composition of which it is a part, unless otherwise indicated.

Herein, "application" and "applied" relate to application to the surface of the human body, in particular the underarm regions, unless the context dictates otherwise.

Herein, features expressed as "preferred" with regard to a particular aspect of the invention should be understood to be preferred with regard to each aspect of the invention.

Herein, preferred, particularly preferred and especially preferred features of the invention are particularly preferred when used in combination with other preferred, particularly preferred and especially preferred features of the invention.

Herein, "ambient conditions" refer to 20°C and 1 atmosphere pressure, unless otherwise indicated.

Herein, the word "comprising" is intended to mean "including" but not necessarily "consisting of" or "composed of." In other words, the listed steps or options need not be exhaustive.

Herein, except in the examples or where otherwise explicitly indicated, all numbers in this description and claims indicating amounts of material or conditions of reaction, physical properties of materials and/or use are to be understood as modified by the word "about".

Herein, numerical ranges expressed in the format "from x to y" are understood to include x and y. When for a specific feature multiple preferred ranges are described in the format "from x to y", it is understood that all ranges combining the different endpoints are also contemplated.

Herein, when materials are referred to as "C14-C22", this means they are selected from one or materials having from 14 to 22 carbon atoms. The same applies to materials referred to as C16-C18, *mutasis mutandis.*

The present invention comprises elements of both packaging and composition of the new deodorant stick product. Because the elements of the packaging are known from the prior art, this detailed description will concentrate on the elements of the deodorant stick composition, although a few packaging elements will be discussed first.

In preferred embodiments, the product has an oval or obround cross-section and especially an oval cross-section.

Deodorant stick compositions used the invention typically do not require a plastic spindle running through them to aid in their elevation from their packaging; indeed, the absence of a central spindle is a preferred feature. The lack of a central spindle can have a detrimental effect of the structural strength of the stick and leads to relatively low preferred ratios of stick height to other dimensions (*vide infra*).

In preferred embodiments, the ratio of the height to the breadth of the cosmetic stick composition is from 1: 2 to 3: 2. It is particularly preferred that this ratio is from 2: 3 to 4: 3. Having the height of the cosmetic stick relative to the breadth of the cosmetic stick within these ranges has been found to enhance the strength of the stick composition and to reduce its tendency to fracture or shear off.

Herein, "stick breadth" is the minimum cross-sectional diameter of the product. For a stick having an oval cross-section, this equates to the minor axis of the oval.

Herein, "stick width" is the cross-sectional diameter of the product measured in a direction orthogonal to the stick breadth.

The ratio of stick breadth to stick width is preferably from 1: 3 to 1: 1 and more preferably from 1: 2 to 1: 1.

Herein, "oval" or "obround" cross-section is the cross-section when viewed from above.

In preferred embodiments, the ratio of the height of the cosmetic stick composition to the height of the holding material is from 1: 2 to 3: 2. It is particularly preferred that this ratio is from 2: 3 to 1: 1.

Deodorant stick compositions used in the invention comprise at least 50% of one or more glycols selected from the group consisting of propylene glycol (PG), propanediol, dipropylene glycol (DPG), butylene glycol, and glycerol. Preferably, the amount of said glycols is from 50 to 90% and more preferably the amount is at least 60%, e.g. 60 to 90%.

Herein, "PG" should be understood to mean 1,2-propanediol. "Propanediol" should be understood to mean 1,3-propanediol.

"Butylene glycol" should be understood to mean 1,3-butanediol.

The content of PG in the deodorant stick composition is preferably from 2 to 15%, more preferably from 3 to 10% and most preferably from 4 to 7.5%.

The content of DPG in the deodorant stick composition is preferably from 20 to 60%, more preferably from 30 to 50% and most preferably from 35 to 45%.

The glycol(s) serve as carrier materials or solvents for the composition. Some may also have desirable sensory properties and/or be humectants (*vide infra*).

Preferred compositions comprise both PG and DPG. In such compositions, the ratio of DPG to PG is preferably from 50: 50 to 95: 5, more preferably from 75: 25 to 92.5: 7.5 and most preferably from 85: 15 to 90: 10.

Preferred compositions comprise glycerol, preferably in an amount of from 5 to 50% and more preferably from 10 to 30% and most preferably from 15 to 25%. Glycerol functions as a humectant, improving the sensory properties of the composition, but can also contribute to the structural quality of the stick, improving its strength without compromising its pay-out.

Preferably, whether present at 50% or more or present at 60% or more, the glycols comprise each of PG, DPG and glycerol. Preferred compositions of this type comprise:
i. from 24 to 55% wt.% of dipropylene glycol;
ii. from 4 to 8 wt.% of propylene glycol;
iii. from 10 to 30 wt.% glycerol; and
iv. from 20 to 40 wt. % of water.

Deodorant stick compositions used with the invention comprise water at from 5 to 40%. Water is a useful carrier material in the composition, although water is not used as the predominant carrier material in order to avoid sensory negatives. Water is preferably present at from 10 to 35% and more preferably at from 20 to 30%.

Deodorant stick compositions used with the invention comprise from 1 to 20 wt.% of sodium salt of one or more C14-C22 fatty acids. Such materials may alternatively be called sodium soaps and serve as structurants for the stick. The level of the sodium soap is preferably from 2 to 15%, more preferably from 3 to 10% and most preferably from 4 to 7.5%.

Preferred sodium soaps are salts of one or more C14-C22 linear fatty acids.

Preferred sodium soaps are salts of one or more C16-C18 linear fatty acids, more preferably they are selected from sodium soaps of palmitic acid and/or stearic acid.

Commercial sodium salts of fatty acids typically have a small amount of free fatty acid associated with them; for example, they may have from 1 to 2% by weight of free fatty acid present. We have found that this can be a problem for refillable deodorant stick compositions since it can detrimentally affect the micro-structure of the stick, resulting in a stick with lesser adhesion to its retaining member.

Herein, "free fatty acid" should be understood as meaning unsaponified fatty acid, i.e. fatty acid that has not been neutralised by base.

In accordance with the first aspect of the present invention, free fatty acid may be minimised by formulating such that deodorant stick composition has a molar ratio of free fatty acid to sodium salt of fatty acid that is less than 1: 99, preferably less than 0.5: 99.5, and more preferably less than 0.1 to 99.9. Hence, the molar content of free fatty acid relative to the total molar content of free fatty acid plus sodium salt thereof, is from 0 to 0.1%, preferably from 0 to 0.5%, and more preferably from 0 to 0.01%.

In accordance with the second aspect of the present invention, free fatty acid may be minimised by adopting a process of manufacture whereby base is added during the manufacturing process of the deodorant stick composition in an amount such that the molar ratio of the base to the sodium salt of a fatty acid is from 2: 98 to 25: 75, preferably from 5: 95 to 20: 80 and more preferably from 5: 95 to 15: 85.

In the process of manufacture referred to in the paragraph immediately above, the base added is preferably an alkaline metal hydroxide, more preferably sodium hydroxide and most preferably an aqueous solution of sodium hydroxide.

The process of manufacture involves heating the components of the deodorant stick composition to a temperature above their melting point with stirring. Base is then added to the mixture in an amount such that molar ratio of the base to the sodium salt of a fatty acid is from 2: 98 to 50: 50, preferably from 5: 95 to 25: 75 and more preferably from 5: 95 to 20: 80, as mentioned above, again with stirring. The molten composition, which is a flowable liquid, is then poured into a mold and placed in contact with the retaining member for the stick. Elements of the retaining member become embedded within the deodorant stick composition. The deodorant stick composition is then cooled to form a solid stick mounted on and attached to the retaining member.

In preferred embodiments, an opacifier may be used to give the deodorant stick composition an aesthetically pleasing visual appearance. A preferred opacifier is a C14-C22 fatty alcohol. Preferably, such opacifiers are linear fatty alcohols, more preferably they are selected from C16-C18 linear fatty alcohols and most preferably the opacifier is stearyl alcohol. Opacifiers are preferably incorporated at from 0.1 to 5% and more preferably at from 0.1 to 2%.

In preferred embodiments, a second structurant may be employed in addition to the sodium salt of C14-C22 fatty acid mentioned above. Such second structurant is preferably used in a lesser amount than the sodium salt of C14-C22 fatty acid; for example, less than 50% of the content of the sodium salt of C14-C22 fatty acid. Such second structurant may be employed at from 0.5 to 10% and preferably at from 1 to 5% of the total composition. Preferred second structurants are poloxamers or poloxamines, in particular poloxamines.

In some preferred embodiments, an additional deodorant active may be incorporated in the deodorant stick composition. The additional deodorant active may be an antimicrobial deodorant, boosting deodorancy by acting against odour-causing bacteria on the skin surface. Preferred additional deodorant actives are organic deodorant actives, in particular natural deodorant actives. Additional deodorant actives may be used at from 0.1 to 10% by weight and preferably at from 0.5 to 5%.

In preferred embodiments, a fragrance or perfume may be incorporated in the deodorant stick composition. Fragrance may be included at from 0.1 to 10%, preferably at from 0.5 to 5%, and more preferably at from 0.5 to 2.5%.

Other components that may be incorporated in the deodorant stick composition include cheating agents, such as EDTA, anti-oxidants, such as BHT, anti-foam agents, such as silicone/hydrated silica, dyes, pigments or colourants.

### Examples

The following examples are intended to clarify the invention and not to limit the invention in any way and specifically not to limit the invention to the examples *per se.*

The following ingredients were used in the examples described hereinbelow.
- Propylene glycol (BASF or Dow Chemical). Pharmacopeia Grade.
- Dipropylene glycol (BASF or Dow Chemical).
- Water, demineralised.
- Stearyl alcohol (Deo Grade) (Hallstar).
- Sodium stearate (Hallstar). Comprised up to 1.3% free fatty acid and was a mixture of C16 and C18 linear fatty acid sodium soaps.
- Sodium hydroxide solution (50 wt.%) (Brenntag).
- Stearic Acid.
- Glycerol (Croda). Glycerin Pharmacopeia Grade. Vege derived.
- Poloxamine 1307 (BASF). Polyoxyethylene polyoxypropylene block copolymer of ethylenediamine.
- Disodium EDTA (BASF).
- Fragrance.

The Examples indicated in Table 1 were prepared in the following manner.

A water bath was set to 90°C. Water and EDTA were added into a vessel. The vessel was placed into the pre-heated water bath and heated to 50°C with stirring.

When the above mixture was at above 50°C, PG, DPG and glycerol were added. The resulting mixture was heated further to 85°C.

Once the mixture had reached 85°C, sodium stearate and stearyl alcohol (where used) were added with stirring and the temperature was maintained until the sodium stearate had dissolved. The water bath was then set to cool.

When the mixture had cooled to 80°C, the Poloxamine 1307 and stearic acid (where used) were added. Mixing continued until the mixture was clear and the temperature had cooled to 75°C.

When the mixture had cooled to 73°C, fragrance and sodium hydroxide solution (where used) were added with stirring. The pouring temperature of the deodorant stick composition precursor was 72 to 74°C. When the precursor had cooled to this temperature, stirring was stopped.

The molten deodorant stick precursor was poured into a mould, placed in contact with the retaining member whilst still molten and then allowed to cool to form solidified stick compositions attached to the retaining member.

**Table 1**

| **Ingredient (wt.%)** | **Sample A** | **Sample 1 (According to invention)** |
|---|---|---|
| PG | 5.5 | 5.5 |
| DPG | 40 | 40 |
| Water | 27.7 | 27.59 |
| Stearyl alcohol | 0 | 0.15 |
| Sodium stearate | 5.5 | 5.5 |
| Sodium hydroxide (50%) | 0 | 0.16 |
| Stearic acid | 0.2 | 0 |
| Glycerol | 18 | 18 |
| Poloxamine 1307 | 2 | 2 |
| Disodium EDTA | 0.1 | 0.1 |
| Fragrance | 1 | 1 |

### Sample Testing

### Bond strength

Bond strength was measured using an Instron apparatus. The stick was held immobile by means of a holding device attached to the retaining member. The stick was held horizontally while force was applied vertically by the apparatus to the side of the stick until breakage occurred. Bond strength was determined as the force in N required to break the stick off the retaining member. Both Sample A and Sample 1 sticks were tested, across multiple replicates (typically 12). The results are shown in the Table 2 below.

### Hardness

Hardness was defined as the depth that a standard cone penetrated a sample under fixed conditions of mass, time and temperature. A PNR 12 Penetrometer (Anton Parr) was used to assess the hardness of the samples as follows.

### Equipment details:

Tapered needle (calibrated), brass/ stainless steel, 2.5g Petrotest Cat.#18-1182.

Plunger: brass, nickel plated, 47.5g Petrotest Cat #18-0042 (ASTM D2884).

### Procedure:

Three replicates of each of the samples were placed in an incubator at 25°C for a minimum of 2 hours. After incubation a cheese wire cutter was used to cut samples at the top to obtain a flat surface, and at the base to remove them from the retaining member. The cut sticks were placed on the platform of the penetrometer, base down and top up. The penetrometer was fitted with the above tapered needle and operated to determine the harness, which was measured as penetration by the needle in mm. The less deep the penetration, the harder the stick. Six measurements at different areas on the surface were taken, three from each side. The results are shown in Table 2 below.

### Pay-out

Pay-out is a measure of the deposition from a stick product in which a device is used for measuring the amount of product a stick pays out for a given amount of force onto a substrate, in this case 230 mm by 75 mm strips of black velvet.

Sample sticks were prepared so that the surface had been evenly cut. Sticks were inserted upside down into the pay-out device by placing the stick into the holder, the grip of which was then tightened to support the stick. The holder assembly was lowered to place the stick samples onto the substrate surface. The deodorant stick samples were applied to the substrate through erosion of the stick's surface by moving the substrate plate backwards and forwards 10 times. An approximately 120mm stripe of product was formed. The process was repeated for a total of five cycles (one forward and one reverse stoke being one cycle). The rate of application of the stick sample to the substrate was controlled by the speed at which the substrate was moved. Five cycles were achieved within a time period of 12 to 15 seconds.

Pay-out was measured by weighing the samples before and after application to the substrate on a 4 decimal place balance and calculating the difference. The results are shown in Table 2 below.

### Opacity

Opacity of the samples was subjectively measured by a panel of trained professionals. Both Sample A and Sample 1 had acceptable opacity.

### Results

**Table 2**

| | **Sample A** | **Sample 1** |
|---|---|---|
| **BOND STRENGTH (N)** | 38 (σ 2.2) | 61 (σ 2.1) |
| **HARDNESS (mm)** | 7.9 | 7.6 |
| **PAY-OUT (g)** | 0.87 | 1.25 |

As can be seen, the bond strength of Sample 1 was significantly greater than that found with Sample A. This means that Sample 1 was much more resilient in terms of the force required to shear it off from its retaining member. Sample 1 was also marginally harder, although this difference was not significant.

In terms of pay-out, this was considerably greater for Sample 1, despite this Sample also binding to its retention member significantly better than Sample A.

## Claims

1. A refillable deodorant stick product comprising:
- a deodorant stick composition mounted on an axially immobile retaining member,
- the retaining member being reversibly connectable to a holder enabling the deodorant stick product to be held in the human hand,
- wherein elements of the retaining member are embedded in the deodorant stick composition,
- wherein the deodorant stick composition comprises:
i. at least 50 wt.% of one or more glycols selected from the group consisting of propylene glycol, propanediol, dipropylene glycol, butylene glycol, glycerol;
ii. 5 to 40 wt.% water;
iii. 1 to 20 wt.% of sodium salt of one or more C14-C22 fatty acids,
**characterised in that** the molar ratio of free fatty acid to sodium salt of fatty acid is less than 1: 99.

2. A deodorant stick product according to claim 1, wherein the deodorant stick composition comprises at least 50 wt.% of a combination of propylene glycol, dipropylene glycol and glycerol.

3. A deodorant stick product according to claim 2, wherein the deodorant stick composition comprises:
i. from 24 to 55% wt.% of dipropylene glycol;
ii. from 4 to 8 wt.% of propylene glycol;
iii. from 10 to 30 wt.% glycerol; and
iv. from 20 to 40 wt. % of water.

4. A deodorant stick product according to any one the preceding claims, wherein the sodium salt of one or more C14-C22 fatty acids is a sodium salt of one or more linear C16-C18 fatty acids.

5. A deodorant stick product according to any one the preceding claims, wherein the molar ratio of free fatty acid to sodium salt of fatty acid in the deodorant stick composition is less than 0.5: 99.5.

6. A deodorant stick product according to any one the preceding claims, wherein the deodorant stick composition comprises from 0.1 to 5 wt.% of a C14 to C22 fatty alcohol.

7. A deodorant stick product according to claim 6, wherein the deodorant stick composition comprises from 0.1 to 2 wt.% of a C16 to C18 fatty alcohol.

8. A deodorant stick product according to any one the preceding claims, wherein the deodorant stick composition comprises less than 0.1wt.% free fatty acid.

9. A deodorant stick product according to any one the preceding claims, wherein the retaining member comprises arcuate bridge elements separated by holes, the bridge structures arcing into the deodorant stick composition and being embedded therein.

10. A deodorant stick product according to any one the preceding claims, wherein the ratio of the height to the breadth of the deodorant stick composition is from 1: 2 to 3: 2.

11. A deodorant stick product according to any one the preceding claims, wherein the product has an oval or obround cross-section when viewed from above.

12. A process for the manufacture of a refillable deodorant stick product, the product comprising:
- a deodorant stick composition mounted on an axially immobile retaining member,
- the retaining member being reversibly connectable to a holder enabling the deodorant stick product to be held in the human hand,
- wherein elements of the retaining member become embedded in the deodorant stick composition;
wherein the process comprises the steps of:
a. combining as a flowable liquid:
(i) at least 50 wt.% of one or more glycols selected from the group consisting of propylene glycol, propanediol, dipropylene glycol, butylene glycol, glycerol;
(ii) 5 to 40 wt.% water;
(iii) 1 to 20 wt.% of sodium salt of one or more C14-C22 fatty acids,
b. adding a base in an amount such that the molar ratio of the base to the sodium salt of a fatty acid is from 2: 98 to 50: 50,
c. pouring the flowable liquid produced in the above steps into a mould such that elements of the retaining member are covered by the flowable liquid, and
d. cooling the flowable liquid such that it forms a solid deodorant stick composition attached to the retention member and with elements of the retention member embedded in the solid deodorant stick composition.

13. A process according to claim 12 for the manufacture of a packaged deodorant stick product according to any of one of claims 1 to 11.

14. A process according to claim 12 or claim 13 wherein the molar ratio of the base to the sodium salt of a fatty acid is from 5: 95 to 20: 80.

15. A process according to any one of claims 12 to claim 14 wherein the base added is an aqueous solution of sodium hydroxide.

## Patentansprüche

1. Nachfüllbares Deodorantstift-Produkt, umfassend:
eine Deodorantstift-Zusammensetzung, die auf einem axial unbeweglichen Halteteil angebracht ist,
wobei das Halteteil reversibel mit einem Halter verbunden werden kann, der es ermöglicht, das Deodorantstift-Produkt in der menschlichen Hand zu halten,
wobei die Elemente des Halteteils in die Deodorantstift-Zusammensetzung eingebettet sind,
wobei die Deodorantstift-Zusammensetzung umfasst:
i. mindestens 50 Gew.-% eines oder mehrerer Glykole, ausgewählt aus der Gruppe, bestehend aus Propylenglykol, Propandiol, Dipropylenglykol, Butylenglykol, Glycerin;
ii. 5 bis 40 Gew.-% Wasser;
iii. 1 bis 20 Gew.-% Natriumsalz einer oder mehrerer C14-C22-Fettsäuren, **dadurch gekennzeichnet, dass** das Molverhältnis von freier Fettsäure zu Natriumsalz der Fettsäure weniger als 1:99 beträgt.

2. Deodorantstift-Produkt nach Anspruch 1, wobei die Deodorantstift-Zusammensetzung mindestens 50 Gew.-% einer Kombination aus Propylenglykol, Dipropylenglykol und Glycerin umfasst.

3. Deodorantstift-Produkt nach Anspruch 2, wobei die Deodorantstift-Zusammensetzung umfasst:
i. 24 bis 55 Gew.-% Dipropylenglykol;
ii. 4 bis 8 Gew.-% Propylenglykol;
iii. 10 bis 30 Gew.-% Glycerin; und
iv. 20 bis 40 Gew.-% Wasser.

4. Deodorantstift-Produkt nach irgendeinem der vorhergehenden Ansprüche, wobei das Natriumsalz einer oder mehrerer C14-C22-Fettsäuren ein Natriumsalz einer oder mehrerer linearer C16-C18-Fettsäuren ist.

5. Deodorantstift-Produkt nach irgendeinem der vorhergehenden Ansprüche, wobei das Molverhältnis von freier Fettsäure zu Natriumsalz der Fettsäure in der Deodorantstift-Zusammensetzung weniger als 0,5:99,5 beträgt.

6. Deodorantstift-Produkt nach irgendeinem der vorhergehenden Ansprüche, wobei die Deodorantstift-Zusammensetzung 0,1 bis 5 Gew.-% eines C14-C22-Fettalkohols umfasst.

7. Deodorantstift-Produkt nach Anspruch 6, wobei die Deodorantstift-Zusammensetzung 0,1 bis 2 Gew.-% eines C16- bis C18-Fettalkohols umfasst.

8. Deodorantstift-Produkt nach irgendeinem der vorhergehenden Ansprüche, wobei die Deodorantstift-Zusammensetzung weniger als 0,1 Gew.-% freie Fettsäure enthält.

9. Deodorantstift-Produkt nach irgendeinem der vorhergehenden Ansprüche, wobei das Halteteil bogenförmige Brückenelemente umfasst, die durch Löcher voneinander getrennt sind, wobei sich die Brückenstrukturen in die Deodorantstift-Zusammensetzung einfügen und darin eingebettet sind.

10. Deodorantstift-Produkt nach irgendeinem der vorhergehenden Ansprüche, wobei das Verhältnis von Höhe zu Breite der Deodorantstift-Zusammensetzung 1:2 bis 3:2 beträgt.

11. Deodorantstift-Produkt nach irgendeinem der vorhergehenden Ansprüche, wobei das Produkt von oben gesehen einen ovalen oder länglich runden Querschnitt aufweist.

12. Verfahren zur Herstellung eines nachfüllbaren Deodorantstift-Produkts, wobei das Produkt umfasst:
eine Deodorantstift-Zusammensetzung, die auf einem axial unbeweglichen Halteteil angebracht ist, wobei das Halteteil reversibel mit einem Halter verbunden werden kann, der es ermöglicht, das Deodorantstift-Produkt in der menschlichen Hand zu halten,
wobei Elemente des Halteteils in die Deodorantstift-Zusammensetzung eingebettet werden;
wobei das Verfahren die folgenden Schritte umfasst:
a. Kombinieren als fließfähige Flüssigkeit:
(i) mindestens 50 Gew.-% eines oder mehrerer Glykole, ausgewählt aus der Gruppe, bestehend aus Propylenglykol, Propandiol, Dipropylenglykol, Butylenglykol und Glycerin;
(ii) 5 bis 40 Gew.-% Wasser;
(iii) 1 bis 20 Gew.-% Natriumsalz einer oder mehrerer C14-C22-Fettsäuren,
b. Zusatz einer Base in einer solchen Menge, dass das Molverhältnis von Base zu Natriumsalz einer Fettsäure zwischen 2:98 und 50:50 liegt,
c. Gießen der in den obigen Schritten hergestellten fließfähigen Flüssigkeit in eine Form, so dass die Elemente des Halteteils von der fließfähigen Flüssigkeit bedeckt sind, und
d. Abkühlen der fließfähigen Flüssigkeit derart, dass sie eine feste Deodorantstift-Zusammensetzung bildet, die an dem Halteteil angebracht ist und bei der Elemente des Halteteils in die feste Deodorantstift-Zusammensetzung eingebettet sind.

13. Verfahren nach Anspruch 12 zur Herstellung eines verpackten Deodorantstift-Produkts nach irgendeinem der Ansprüche 1 bis 11.

14. Verfahren nach Anspruch 12 oder Anspruch 13, wobei das Molverhältnis von Base zu Natriumsalz einer Fettsäure 5:95 bis 20:80 beträgt.

15. Verfahren nach irgendeinem der Ansprüche 12 bis 14, wobei die zugesetzte Base eine wässrige Lösung von Natriumhydroxid ist.

## Revendications

1. Produit en stick déodorant rechargeable comprenant :
- une composition de stick déodorant montée sur un élément de retenue axialement immobile,
- l'élément de retenue pouvant être relié de manière réversible à un support permettant au produit en stick déodorant d'être tenu dans la main humaine,
- dans lequel des éléments de l'élément de retenue sont incorporés dans la composition de stick déodorant,
- dans lequel la composition de stick déodorant comprend :
i. au moins 50 % en poids d'un ou de plusieurs glycols choisis dans le groupe constitué par le propylène glycol, le propanediol, le dipropylène glycol, le butylène glycol, le glycérol ;
ii. de 5 à 40 % en poids d'eau ;
iii. de 1 à 20 % en poids de sel de sodium d'un ou de plusieurs acides gras en C14 à C22,
**caractérisé en ce que** le rapport molaire de l'acide gras libre au sel de sodium d'acide gras est inférieur à 1:99.

2. Produit en stick déodorant selon la revendication 1, dans lequel la composition de stick déodorant comprend au moins 50 % en poids d'une combinaison de propylène glycol, de dipropylène glycol et de glycérol.

3. Produit en stick déodorant selon la revendication 2, dans lequel la composition de stick déodorant comprend :
i. de 24 à 55 % en poids de dipropylène glycol ;
ii. de 4 à 8 % en poids de propylène glycol ;
iii. de 10 à 30 % en poids de glycérol ; et
iv. de 20 à 40 % en poids d'eau.

4. Produit en stick déodorant selon l'une quelconque des revendications précédentes, dans lequel le sel de sodium d'un ou de plusieurs acides gras en C14 à C22 est un sel de sodium d'un ou plusieurs acides gras en C16 à C18 linéaires.

5. Produit en stick déodorant selon l'une quelconque des revendications précédentes, dans lequel le rapport molaire de l'acide gras libre au sel de sodium d'acide gras dans la composition de stick déodorant est inférieur à 0,5:99,5.

6. Produit en stick déodorant selon l'une quelconque des revendications précédentes, dans lequel la composition de stick déodorant comprend de 0,1 à 5 % en poids d'un alcool gras en C14 à C22.

7. Produit en stick déodorant selon la revendication 6, dans lequel la composition de stick déodorant comprend de 0,1 à 2 % en poids d'un alcool gras en C16 à C18.

8. Produit en stick déodorant selon l'une quelconque des revendications précédentes, dans lequel la composition de stick déodorant comprend moins de 0,1 % en poids d'acide gras libre.

9. Produit en stick déodorant selon l'une quelconque des revendications précédentes, dans lequel l'élément de retenue comprend des éléments de pont arqués séparés par des trous, les structures de pont s'arquant dans la composition de stick déodorant et étant incorporées dans celle-ci.

10. Produit en stick déodorant selon l'une quelconque des revendications précédentes, dans lequel le rapport de la hauteur à la largeur de la composition de stick déodorant est de 1:2 à 3:2.

11. Produit en stick déodorant selon l'une quelconque des revendications précédentes, dans lequel le produit a une section transversale ovale ou oblongue lorqu'obversé de dessus.

12. Procédé de fabrication d'un produit en stick déodorant rechargeable, le produit comprenant :
- une composition de stick déodorant montée sur un élément de retenue axialement immobile,
- l'élément de retenue pouvant être relié de manière réversible à un support permettant au produit en stick déodorant d'être tenu dans la main humaine,
- dans lequel des éléments de l'élément de retenue sont incorporés dans la composition de stick déodorant ;
dans lequel le procédé comprend les étapes consistant à :
a. combiner sous forme de liquide fluide :
(i) au moins 50 % en poids d'un ou de plusieurs glycols choisis dans le groupe constitué par le propylène glycol, le propanediol, le dipropylène glycol, le butylène glycol et le glycérol ;
(ii) de 5 à 40 % en poids d'eau ;
(iii) de 1 à 20 % en poids de sel de sodium d'un ou de plusieurs acides gras en C14 à C22,
b. ajouter une base en une quantité telle que le rapport molaire de la base au sel de sodium d'un acide gras est de 2:98 à 50:50,
c. verser le liquide fluide produit lors des étapes précédentes dans un moule de telle sorte que les éléments de l'élément de retenue sont recouverts par le liquide fluide, et
d. refroidir le liquide fluide de telle sorte qu'il forme une composition de stick déodorant solide fixée à l'élément de retenue et dont les éléments de rétention sont incorporés dans la composition de stick déodorant solide.

13. Procédé selon la revendication 12 pour la fabrication d'un produit en stick déodorant conditionné selon l'une quelconque des revendications 1 à 11.

14. Procédé selon la revendication 12 ou la revendication 13 dans lequel le rapport molaire de la base au sel de sodium d'un acide gras est de 5:95 à 20:80.

15. Procédé selon l'une quelconque des revendications 12 à 14, dans lequel la base ajoutée est une solution aqueuse d'hydroxyde de sodium.
